(19) Europäisches Patentamt
European Patent Office
Office européen des brevets

(11) Veröffentlichungsnummer: **0 052 300**
**B1**

(12) # EUROPÄISCHE PATENTSCHRIFT

(45) Veröffentlichungstag der Patentschrift: **13.02.85**

(51) Int. Cl.[4]: **C 07 D 211/90,** A 61 K 31/44, C 07 D 401/14, C 07 D 405/14, C 07 D 409/14, C 07 D 413/14, C 07 D 417/14

(21) Anmeldenummer: **81109456.4**

(22) Anmeldetag: **31.10.81**

(54) **C-3 verknüpfte 1,4-Dihydropyridine, ihre Verwendung in Arzneimitteln und Verfahren zu ihrer Herstellung.**

(30) Priorität: **13.11.80 DE 3042769**

(43) Veröffentlichungstag der Anmeldung: **26.05.82 Patentblatt 82/21**

(45) Bekanntmachung des Hinweises auf die Patenterteilung: **13.02.85 Patentblatt 85/7**

(84) Benannte Vertragsstaaten: **AT BE CH DE FR GB IT LI LU NL SE**

(56) Entgegenhaltungen:
**DE - A - 1 795 791**
**DE - A - 2 117 571**
**DE - A - 2 847 236**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Goldmann, Siegfried, Dr., Kuckuckstrasse 41, D-5600 Wuppertal 2 (DE)**
Erfinder: **Knorr, Andreas, Dr., Pahlkestrasse 15, D-5600 Wuppertal 1 (DE)**

**EP 0 052 300 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die Erfindung betrifft neue dimere 1,4-Dihydropyridine, die in 3-Position miteinander verknüpft sind, mehrere Verfahren zu ihrer Herstellung sowie ihre Verwendung in Arzneimitteln, insbesondere in Arzneimitteln mit kreislaufbeeinflussender Wirkung.

Es ist bereits bekannt, daß bestimmte 1,4-Dihydropyridin-Derivate interessante pharmakologische Eigenschaften aufweisen [vgl. F. Bossert et al., Naturwissenschaften 58, 578 (1971) und DE-OS 2 117 571].

Die Erfindung betrifft neue C-3 verknüpfte 1,4-Dihydropyridine der allgemeinen Formel I

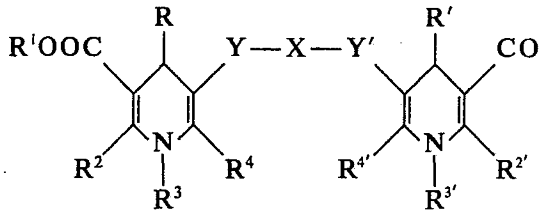

(I)

in welcher

R und R' gleich oder verschieden sind und jeweils für einen Phenylrest oder für einen Thienyl-, Furyl- oder Pyridylrest stehen, wobei der Phenylrest gegebenenfalls ein oder zwei gleiche oder verschiedene Substituenten aus der Gruppe Nitro, Cyano, Acido, Halogen, Trifluormethyl, Hydroxy, Amino, Alkyl, Alkoxy, Alkylamino oder Alkylmercapto mit jeweils 1 oder 2 Kohlenstoffatomen in den Alkylgruppen, substituiert ist,

$R^1$ und $R^{1'}$ gleich oder verschieden sind, jeweils für einen geradkettigen oder verzweigten Kohlenwasserstoffrest mit bis zu 6 Kohlenstoffatomen stehen, der gegebenenfalls durch ein Sauerstoffatom in der Kette unterbrochen ist und der gegebenenfalls substituiert ist durch Fluor, Chlor, Hydroxy, Phenyl oder Phenoxy,

$R^2$, $R^{2'}$, $R^4$ und $R^{4'}$ gleich oder verschieden sind und jeweils für Wasserstoff, geradkettigen oder verzweigten Alkylrest mit bis zu 4 Kohlenstoffatomen stehen, der gegebenenfalls durch Fluor, Chlor, Hydroxy, Phenyl oder Amino substituiert ist,

$R^3$ und $R^{3'}$ gleich oder verschieden sind und jeweils für Wasserstoff, Alkyl mit 1 bis 4 Kohlenstoffatomen, Phenyl, Benzyl oder Phenethyl, gegebenenfalls substituiert durch Hydroxy, Fluor oder Chlor,

Y und Y' jeweils gleich oder verschieden sind und die Bedeutung $-COO-$, $-CONH-$, $-CO-$ oder $-SO_2-$ haben, und

X ein Brückenglied bedeutet, das aus einer bis zehn $CH_2$-Gruppen als Kettenglied besteht, wobei keine dieser $CH_2$-Gruppen ein Ringglied sein darf, und wobei dieses Brückenglied zusätzlich in beliebiger Reihenfolge 1 bis 3 gleiche oder verschiedene Kettenglieder aus der Gruppe O, S, CO, CS, $NR^5$, $C(R^6)_2$, $CH=N$, Phenylen, Naphthylen, Pyrrylen, Cycloalkylen mit 5 bis 7 Kohlenstoffatomen, Piperazinylen, Piperidinylen, Pyrrolidinylen oder Morpholinylen enthalten kann, wobei $R^5$ für Wasserstoff, Benzyl oder Alkyl mit 1 bis 4 Kohlenstoffatomen steht und $R^6$ für Wasserstoff, Benzyl, Phenyl, Fluor, Chlor, Alkyl mit 1 bis 4 Kohlenstoffatomen, Hydroxy, Trifluormethyl, Cyano, Carboxy oder Amino steht,

sowie ihre pharmakologisch unbedenklichen Säureadditionssalze.

Die erfindungsgemäßen Verbindungen der allgemeinen Formel I können erhalten werden, indem man

A) 1,4-Dihydropyridinhydroxyderivate der allgemeinen Formel II

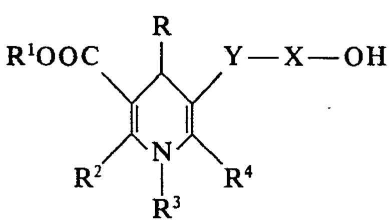

(II)

in welcher

R, $R^1$, $R^2$, $R^3$, $R^4$, Y und X die oben angegebene Bedeutung haben,

in einem inerten organischen Lösungsmittel in Gegenwart von wasserbindenden Mitteln mit 1,4-Dihydropyridin-3-carbonsäurederivaten der allgemeinen Formel III

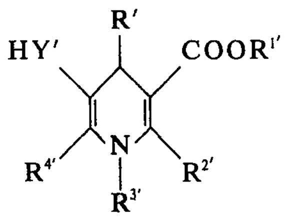

(III)

in welcher
$R'$, $R^{1'}$, $R^{2'}$, $R^{3'}$, $R^{4'}$ und $Y'$ die oben angegebene Bedeutung besitzen, wobei $Y'$ nicht für eine Carbonylgruppe steht,
in äquivalenten Mengen unter Wasserabspaltung bei Temperaturen zwischen 0 und 180°C umsetzt, oder

B) 1,4-Dihydropyridincarbonsäuren der allgemeinen Formel IV

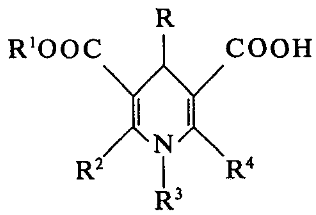

(IV)

in welcher
R, $R^1$, $R^2$, $R^3$ und $R^4$ die oben angegebene Bedeutung haben,
in Gegenwart eines inerten organischen Lösungsmittels und gegebenenfalls in Gegenwart eines Kondensationsmittels mit einer bifunktionellen Verbindung der allgemeinen Formel V

$$Z—X—Z' \qquad (V)$$

in welcher
X die oben angegebene Bedeutung hat und
Z und Z′ jeweils gleich oder verschieden sind und für Hydroxyl, Mercapto oder den Rest $NR^5$ stehen, wobei $R^5$ die oben angegebene Bedeutung besitzt,
in einem molaren Verhältnis von etwa 2:1 bei Temperaturen zwischen 0 und 180°C umsetzt, wobei nach dieser Variante nur Verbindungen der allgemeinen Formel I erhalten werden, in denen Y und Y′ nicht für eine Carbonylgruppe stehen, oder

C) Yliden-β-ketoester der allgemeinen Formel VI

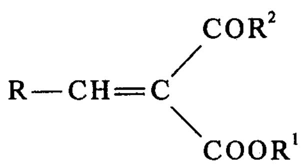

(VI)

R, $R^1$ und $R^2$ die oben angegebene Bedeutung haben,
mit Enaminocarbonsäureestern der allgemeinen Formel VII

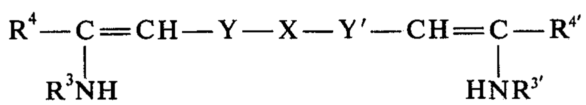

(VII)

in welcher
$R^3$, $R^{3'}$, $R^4$, $R^{4'}$, Y und Y′ die oben angegebene Bedeutung haben,
in Gegenwart von inerten organischen Lösungsmitteln in molaren Verhältnissen von etwa 2:1 bei Temperaturen zwischen 0 und 180°C umsetzt.

Nach dieser Verfahrensvariante C) lassen sich vorzugsweise symmetrische Verbindungen, d. h. Verbindungen in denen jeweils Y und Y′, $R^3$ und $R^{3'}$ und $R^4$ und $R^{4'}$ identisch sind, herstellen. Nach dieser Verfahrensvariante C) lassen sich auch symmetrische Verbindungen der allgemeinen Formel I, in welcher Y eine Carbonylgruppe bedeutet, herstellen.

Je nach Art der eingesetzten Ausgangsstoffe kann die Synthese der erfindungsgemäßen Verbindungen der allgemeinen Formel I nach den einzelnen Verfahrensvarianten durch folgendes Formelschema wiedergegeben werden:

Verfahrensvariante A

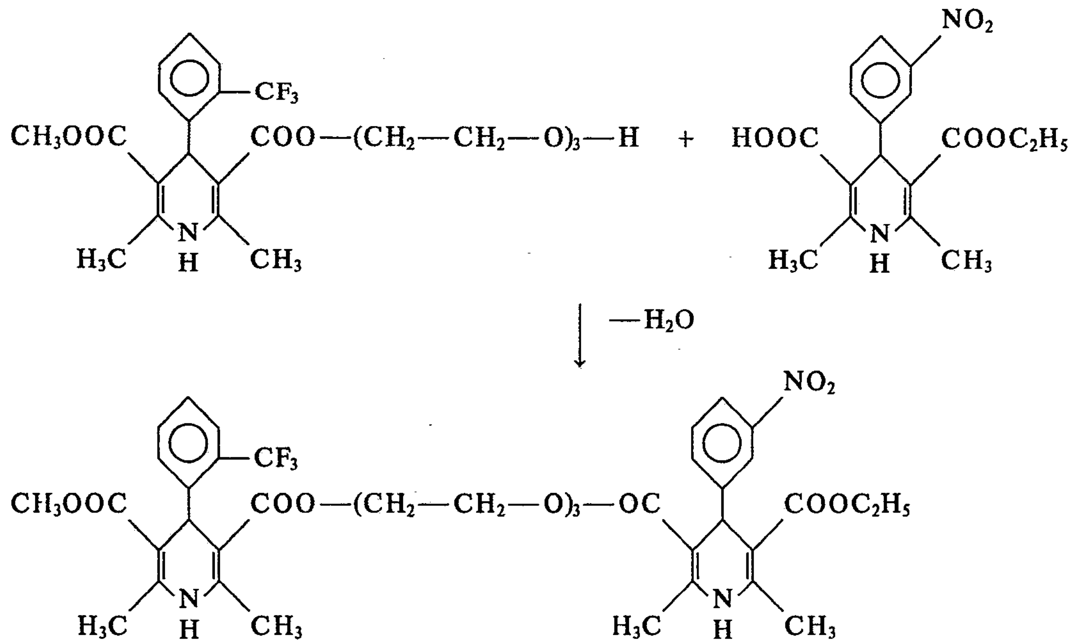


Verfahrensvariante B

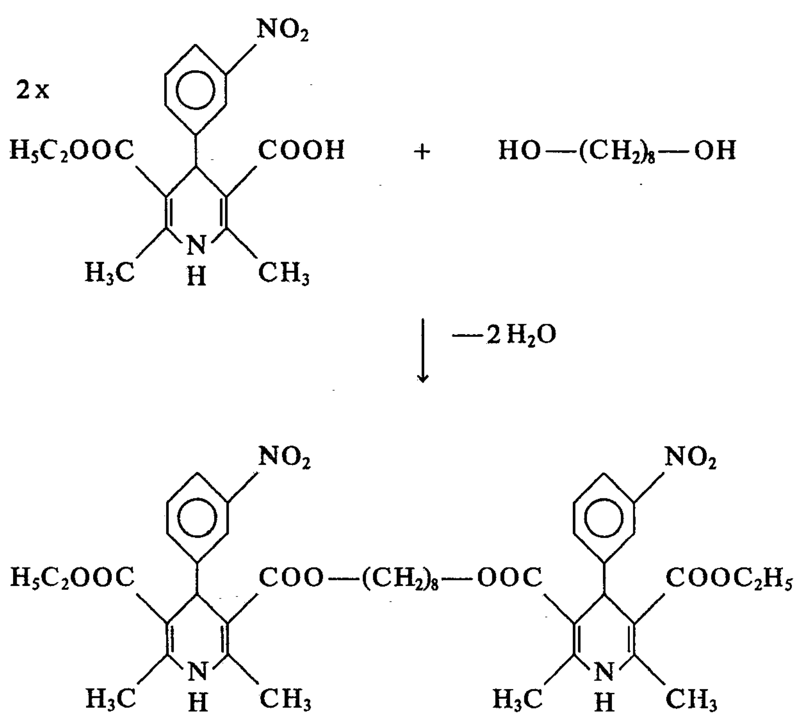

Verfahrensvariante C

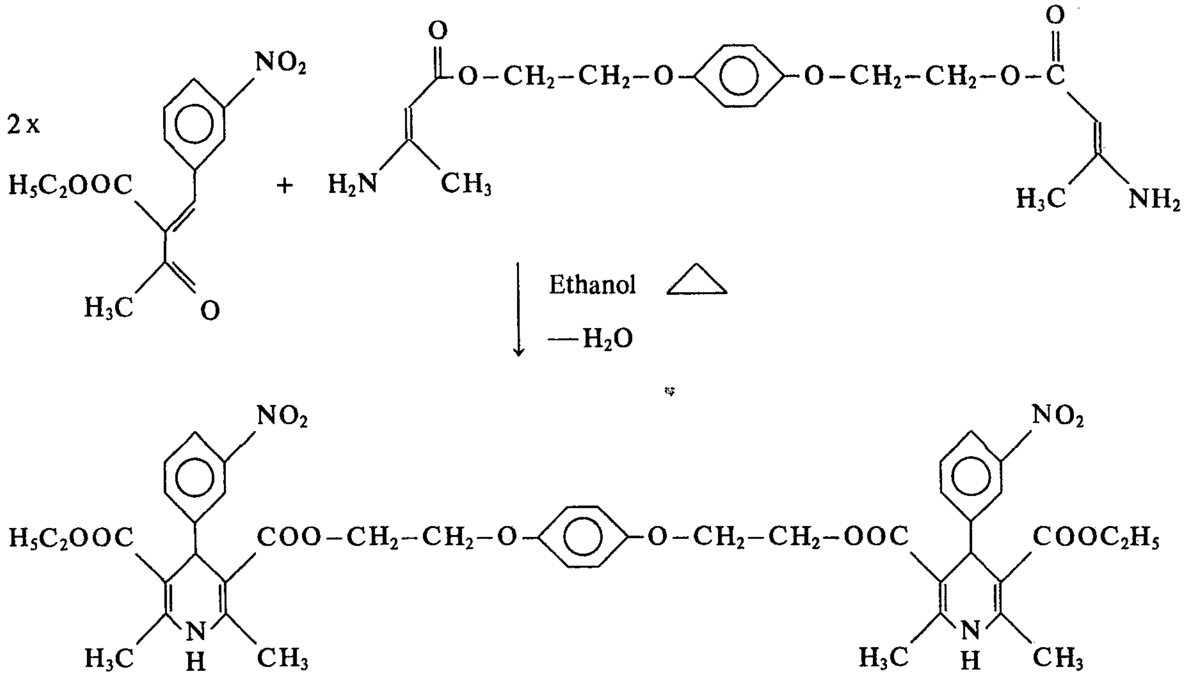


Die als Ausgangsstoffe verwendeten 1,4-Dihydropyridinhydroxyderivate der allgemeinen Formel II und die 1,4-Dihydropyridin-3-carbonsäurederivate der allgemeinen Formel III sind bekannt oder können nach bekannten Methoden hergestellt werden (vgl. DE-OS 2 117 571).

Die 1,4-Dihydropyridincarbonsäuren der allgemeinen Formel IV sind bekannt oder können nach bekannten Methoden hergestellt werden (vgl. EP-A-0 011 706).

Die bifunktionellen Verbindungen der allgemeinen Formel V sind ebenfalls bekannt bzw. können nach bekannten Methoden hergestelllt werden (vgl. Beilstein, Band I, 464—502).

Die Yliden-$\beta$-ketoester der allgemeinen Formel VI sind bekannt oder können nach literaturbekannten Verfahren hergestellt werden [vgl. G. Jones, The »Knoevenagel-Condensation«, in Org. Reactions Vol. XV, 204ff. (1967)].

Die als Ausgangsstoffe verwendeten Enaminocarbonsäureester der allgemeinen Formel VII sind bekannt oder können ebenfalls nach literaturbekannten Methoden hergestellt werden [vgl. A. C. Cope, J. Am. Chem. Soc. 67, 1017 (1945)].

Als Verdünnungsmittel zur Verwendung in den Verfahrensvarianten A und B kommen alle aprotischen organischen Lösungsmittel in Frage. Hierzu gehören vorzugsweise Ether wie Dioxan, Diethylether, Tetrahydrofuran, Glykoldimethylether oder Kohlenwasserstoffe wie Benzol, Toluol, Xylol oder Dimethylformamid, Dimethylsulfoxid, Acetonitril, Pyridin oder Hexamethylphosphorsäurediamid.

Im Falle der Verfahrensvariante C können außerdem noch Alkohole, z. B. Methanol, Ethanol oder Isopropanol vorteilhaft eingesetzt werden.

Als wasserbindende Mittel (Verfahrensvarianten A und C) können alle hierfür üblichen Reagentien verwendet werden, insbesondere ist die Verwendung von Dicyclohexylcarbodiimid und der Zusatz eines Katalysators wie z. B. 4-Dimethylaminopyridin, sehr vorteilhaft.

Die Reaktionstemperaturen können in einem größeren Bereich variiert werden. Im allgemeinen arbeitet man zwischen 0 und 180°C, vorzugsweise zwischen 20 und 120°C.

Die Umsetzungen können bei Normaldruck oder auch bei erhöhtem Druck durchgeführt werden. Im allgemeinen arbeitet man bei Normaldruck.

Die erfindungsgemäßen Verbindungen der allgemeinen Formel I besitzen wertvolle pharmakologische Eigenschaften. Aufgrund ihrer kreislaufbeeinflussenden Wirkung können sie als antihypertensive Mittel, als Vasodilatatoren, als Zerebraltherapeutika und als Coronartherapeutika Verwendung finden. Besonders hervorgehoben sei ihre langanhaltende Wirkung, welche die Applikationshäufigkeit bei der Behandlung von Kreislauferkrankungen reduziert. Sie stellen somit eine Bereicherung der Pharmazie dar.

Im einzelnen seien folgende Hauptwirkungen genannt:

1. Die Verbindungen bewirken bei parenteraler, oraler und perlingualer Zugabe eine deutliche und langanhaltende Erweiterung der Coronargefäße. Diese Wirkung auf die Coronargefäße wird durch einen gleichzeitigen nitritähnlichen herzentlastenden Effekt verstärkt.
   Sie beeinflussen bzw. verändern den Herzstoffwechsel im Sinne einer Energieersparnis.

2. Die Erregbarkeit des Reizbildungs- und Erregungsleitungssystems innerhalb des Herzens wird herabgesetzt, so daß eine in therapeutischen Dosen nachweisbare Antiflimmerwirkung resultiert.
3. Der Tonus der glatten Muskulatur der Gefäße wird unter der Wirkung der Verbindungen stark vermindert. Diese gefäßspasmolytische Wirkung kann im gesamten Gefäßsystem stattfinden, oder sich mehr oder weniger isoliert in umschriebenen Gefäßgebieten (wie z. B. dem Zentralnervensystem) manifestieren.
4. Die Verbindungen senken den Blutdruck von normotonen und hypertonen Tieren und können somit als antihypertensive Mittel verwendet werden.
5. Die Verbindungen haben stark muskulär-spasmolytische Wirkungen, die an der glatten Muskulator des Magens, Darmtraktes, des Urogenitaltraktes und des Respirationssystems deutlich werden.

Die neuen Wirkstoffe können in bekannter Weise in die üblichen Formulierungen übergeführt werden, wie Tabletten, Kapseln, Dragees, Pillen, Granulate, Aerosole, Sirupe, Emulsionen, Suspensionen und Lösungen, unter Verwendung inerter, nicht-toxischer, pharmazeutisch geeigneter Trägerstoffe oder Lösungsmittel. Hierbei soll die therapeutisch wirksame Verbindung jeweils in einer Konzentration von etwa 0,5 bis 90 Gew.-% der Gesamtmischung vorhanden sein, d. h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z. B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt:

Wasser, nicht-toxische organische Lösungsmittel, wie Paraffine (z. B. Erdölfraktionen), pflanzliche Öle (z. B. Erdnuß-/Sesamöl), Alkohole (z. B. Ethylalkohol, Glycerin), Glykole (z. B. Propylenglykol, Polyethylenglykol), feste Trägerstoffe, wie z. B. natürliche Gesteinsmehle (z. B. Kaoline, Tonerden, Talkum, Kreide), synthetische Gesteinsmehle (z. B. hochdisperse Kieselsäure, Silikate), Zucker (z. B. Roh-, Milch- und Traubenzucker), Emulgiermittel (z. B. Polyoxyethylen-Fettsäure-Ester, Polyoxyethylen-Fettalkohol-Ether, Alkylsulfonate und Arylsulfonate), Dispergiermittel (z. B. Lignin, Sulfitablaugen, Methylcellulose, Stärke und Polyvinylpyrrolidon) und Gleitmittel (z. B. Magnesiumstearat, Talkum, Stearinsäure und Natriumlaurylsulfat).

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen und/oder Elixiere, die für orale Anwendungen gedacht sind, können die Wirkstoffe außer den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,01 bis 10 mg/kg, vorzugsweise etwa 0,05 bis 5 mg/kg Körpergewicht pro Tag zur Erzielung wirksamer Ergebnisse zu verabreichen, und bei oraler Applikation beträgt die Dosierung etwa 0,05 bis 20 mg/kg, vorzugsweise 0,5 bis 5 mg/kg Körpergewicht pro Tag.

Trotzdem kann es gegebenenfalls erforderlich sein, von den genannten Mengen abzuweichen, und zwar in Abhängigkeit vom Körpergewicht des Versuchstieres bzw. der Art des Applikationsweges, aber auch aufgrund der Tierart und deren individuellem Verhalten gegenüber dem Medikament bzw. deren Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehrere Einzelgaben über den Tag zu verteilen. Für die Applikation in der Humanmedizin ist der gleiche Dosierungsspielraum vorgesehen. Sinngemäß gelten hierbei auch die obigen Ausführungen.

Beispiel 1

[2,6-Dimethyl-5-ethoxycarbonyl-4-(3-nitrophenyl)-1,4-dihydropyridin-3-carbonsäure]-[2,6-dimethyl-5-methoxycarbonyl-4-(2-chlorphenyl)-1,4-dihydropyridin-3-carbonsäure]-1,4-butandiylester

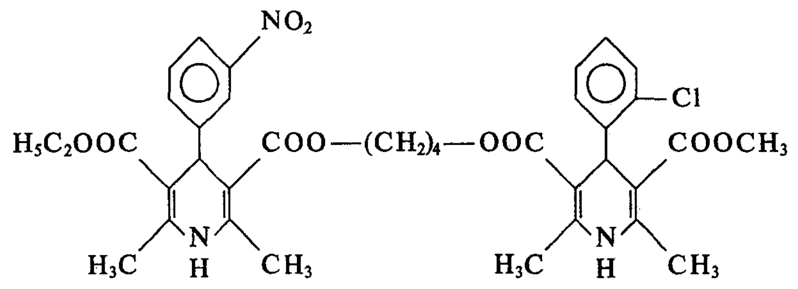

Verfahrensvariante A

25 mmol 2,6-Dimethyl-5-(4-hydroxybutoxy)-carbonyl-4-(3-nitrophenyl)-1,4-dihydropyridin-3-carbonsäureethylester werden zusammen mit 25 mmol Dicyclohexylcarbodiimid und 25 mmol 2,6-Dimethyl-5-methoxycarbonyl-4-(2-chlorphenyl)-1,4-dihydropyridin-3-carbonsäure in 50 ml wasserfreiem Dimethylformamid gelöst und unter Zusatz von 0,2 g 4-Dimethylaminopyridin 4 Stunden auf 100°C erhitzt. Anschließend wird abgesaugt, das Filtrat mit Methylenchlorid verdünnt und jeweils mit wäßriger NaOH und HCl ausgeschüttelt, getrocknet und einrotiert.
Anschließend wird an Kieselgel mit Ether chromatographiert.
Ausbeute: 25%, amorpher Schaum.

$^1$H-NMR:
$\delta$ = 1,2 (t, 3H), 1,4—1,8 (m, 4H), 2,4 (s, 12H), 3,6 (s, 3H), 3,9—4,4 (m, 6H), 5,2 (s, 1H), 5,4 (s, 1H), 5,8 (s, NH), 6,3 (s, NH), 6,9—8,3 (m, 8H).

Beispiel 2

Bis-[2,6-dimethyl-5-methoxycarbonyl-4-(3-nitrophenyl)-1,4-dihydropyridin-3-carbonsäure]-1,6-hexandiylester

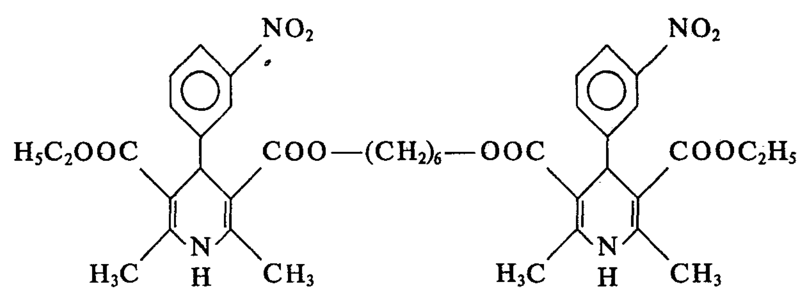

Verfahrensvariante B

50 mmol 2,6-Dimethyl-5-methoxycarbonyl-4-(3-nitrophenyl)-1,4-dihydropyridin-3-carbonsäure werden zusammen mit 50 mmol Dicyclohexylcarbodiimid und 25 mmol Hexandiol-1,6 in 50 ml wasserfreiem Dimethylformamid gelöst und unter Zusatz von 0,2 g 4-Dimethylaminopyridin 4 Stunden bei 100°C gerührt. Anschließend wird abgesaugt, das Filtrat mit Methylenchlorid verdünnt und jeweils mit wäßrigem NaOH und HCl ausgeschüttelt, getrocknet einrotiert und aus Methanol umkristallisiert.
Schmelzpunkt: 177—179°C; Ausbeute: 37%.

Beispiel 3

Bis-[2,6-dimethyl-5-methoxycarbonyl-4-(2-chlorphenyl)-1,4-dihydropyridin-3-carbonsäure]-1,3-propandiylester

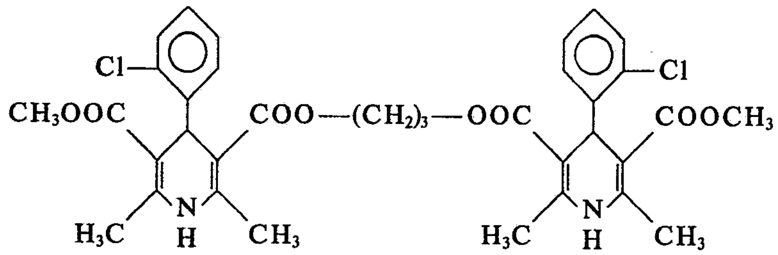

Verfahrensvariante C

25 mmol Bis-(3-aminocrotonsäure)-1,3-propandiylester werden zusammen mit 50 mmol 2-Chlorbenzylidenacetessigsäuremethylester in 100 ml absolutem Ethanol 14 Stunden unter $N_2$ am Rückfluß gekocht.

Nach dem Erkalten wird das Lösungsmittel im Vakuum abdestilliert und mit 50%igem wäßrigem Ethanol aufgenommen. Der halbfeste Rückstand wurde aus Methanol umkristallisiert.

Schmelzpunkt: 200 bis 203°C; Ausbeute: 50%.

Beispiel 4

Bis-[2,6-dimethyl-5-methoxycarbonyl-4-(3-nitrophenyl)-1,4-dihydropyridin-3-carbonsäure]-triethylenglykoldiester

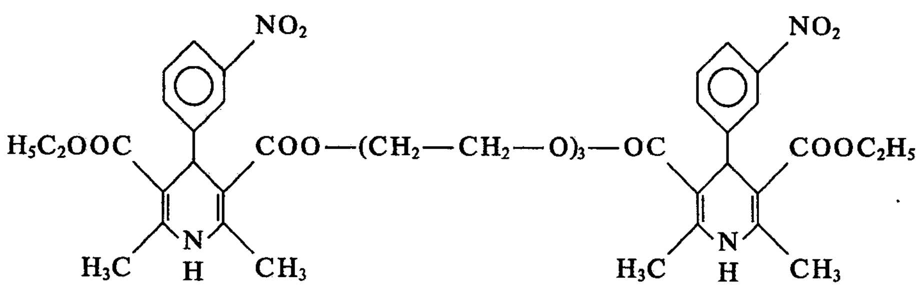

100 mmol 2,6-Dimethyl-5-ethoxycarbonyl-4-(3-nitrophenyl)-1,4-dihydropyridin-3-carbonsäure werden analog Beispiel 2 mit 50 mmol Triethylenglykol umgesetzt und aufgearbeitet.

Schmelzpunkt: 113 bis 120°C; Ausbeute: 32%.

Beispiel 5

Bis-[2,6-dimethyl-5-ethoxycarbonyl-4-(3-nitrophenyl)-1,4-dihydropyridin-3-carbonsäure]-1,10-decandiylester

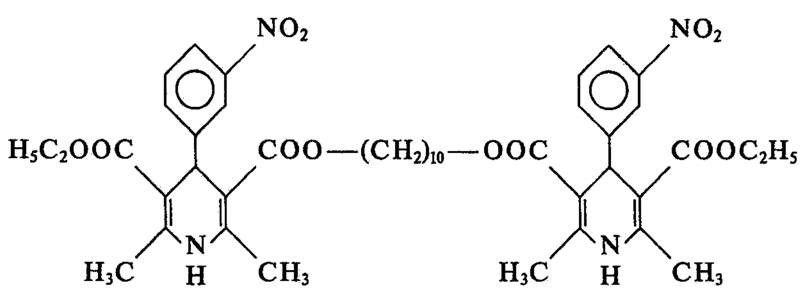

100 mmol 2,6-Dimethyl-5-ethoxycarbonyl-4-(3-nitrophenyl)-1,4-dihydropyridin-3-carbonsäure werden mit 50 mmol 1,10-Decandiol analog Beispiel 2 umgesetzt.
Schmelzpunkt: 121 bis 125°C; Ausbeute: 35%.

Beispiel 6

Bis-[2,6-dimethyl-5-ethoxycarbonyl-4-(3-nitrophenyl)-1,4-dihydropyridin-3-carbonsäure]-1,8-octandiylester

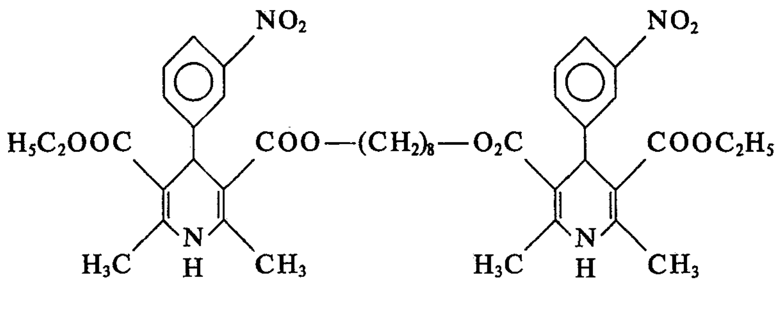

100 mmol 2,6-Dimethyl-5-ethoxycarbonyl-4-(3-nitrophenyl)-1,4-dihydropyridin-3-carbonsäure werden mit 50 mmol 1,8-Octandiol analog Beispiel 2 umgesetzt.
Schmelzpunkt: 170 bis 184°C; Ausbeute: 47%.

Beispiel 7

Bis-[2,6-dimethyl-5-ethoxycarbonyl-4-(3-nitrophenyl)-1,4-dihydropyridin-3-carbonsäure]-1,5-pentandiylester

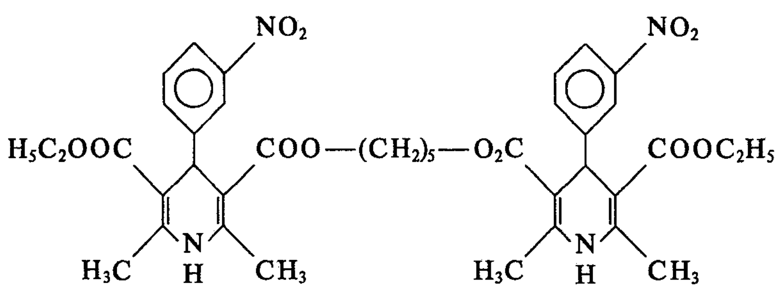

100 mmol 2,6-Dimethyl-5-ethoxycarbonyl-4-(3-nitrophenyl)-1,4-dihydropyridin-3-carbonsäure werden mit 50 mmol 1,5-Pentandiol analog Beispiel 2 umgesetzt.
Schmelzpunkt: 145°C; Ausbeute: 11%.

Beispiel 8

Bis-[2,6-dimethyl-5-ethoxycarbonyl-4-(3-nitrophenyl)-1,4-dihydropyridin-3-carbonsäure]-1,4-bis-(2-hydroxyethoxy)-benzol-diester

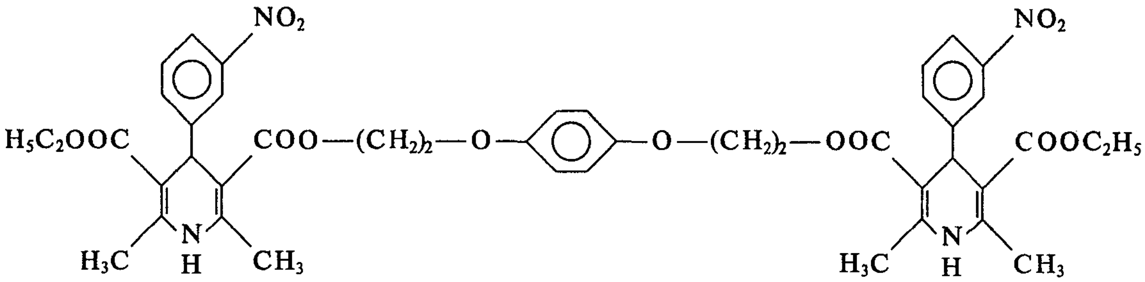

100 mmol 2,6-Dimethyl-5-ethoxycarbonyl-4-(3-nitrophenyl)-1,4-dihydropyridin-3-carbonsäure werden mit 50 mmol 1,4-Bis-(2-hydroxyethoxy)-benzol analog Beispiel 2 umgesetzt.
Schmelzpunkt: 160 bis 190°C (Zers.); Ausbeute: 49%.

Beispiel 9

Bis-[2,6-dimethyl-5-ethoxycarbonyl-4-(3-nitrophenyl)-1,4-dihydropyridin-3-carbonsäure]-tetraethylenglykol-diester

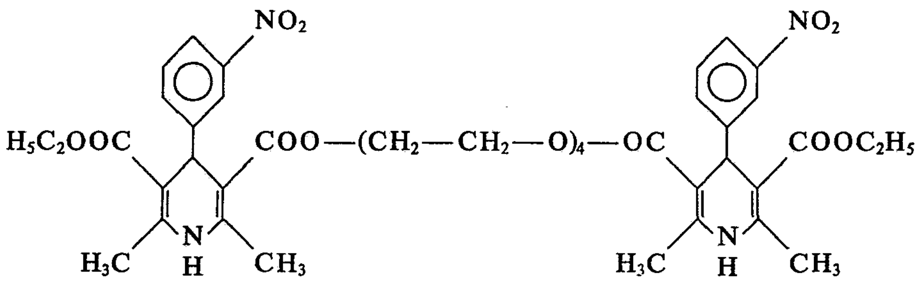

100 mmol 2,6-Dimethyl-5-ethoxycarbonyl-4-(3-nitrophenyl)-1,4-dihydropyridin-3-carbonsäure werden analog Beispiel 2 mit 50 mmol Tetraethylenglykol umgesetzt und aufgearbeitet.
Schmelzpunkt: 93 bis 98°C; Ausbeute: 16%.

Beispiel 10

Bis-[2,6-dimethyl-5-isopropoxycarbonyl-4-(3-nitrophenyl)-1,4-dihydropyridin-3-carbonsäure]-1,6-hexandiylester

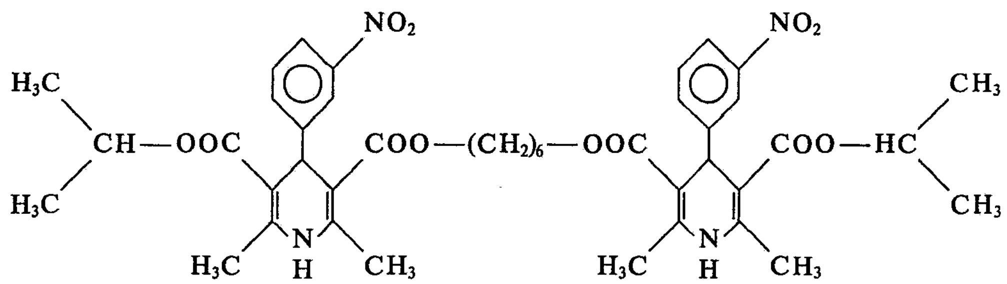

25 mmol Bis-(3-aminocrotonsäure)-1,6-hexandiylester werden mit 50 mmol 3-Nitrobenzylidenacetessigsäurediisopropylester analog Beispiel 3 umgesetzt.
Schmelzpunkt: 120–136°C; Ausbeute: 83%.

Beispiel 11

Bis-[2,6-dimethyl-5-methoxycarbonyl-4-(2-nitrophenyl)-1,4-dihydropyridin-3-carbonsäure]-1,6-hexandiylester

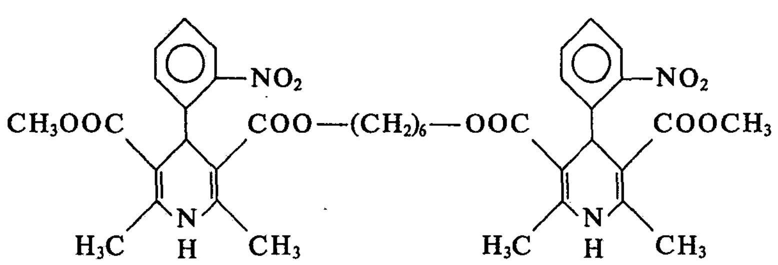

25 mmol Bis-(3-aminocrotonsäure)-1,6-hexandiylester werden mit 50 mmol 2-Nitrobenzylidenacetessigsäuremethylester analog Beispiel 3 umgesetzt.
Schmelzpunkt: 88—95°C; Ausbeute: 82%.

Beispiel 12

Bis-[2,6-dimethyl-5-methoxycarbonyl-4-(2-chlorphenyl)-1,4-dihydropyridin-3-carbonsäure]-hexandiylester

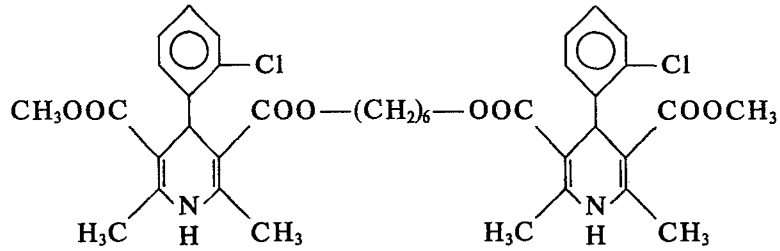

25 mmol Bis-(3-aminocrotonsäure)-1,6-hexandiylester werden mit 50 mmol 2-Chlorbenzylidenacetessigsäuremethylester analog Beispiel 3 umgesetzt.
Schmelzpunkt: 152—158°C; Ausbeute: 27%.

Beispiel 13

Bis-[2,6-dimethyl-5-ethoxycarbonyl-4-(3-nitrophenyl)-1,4-dihydropyridin-3-carbonsäure]-N,N'-bis-(2-hydroxyethyl)-piperazindiester

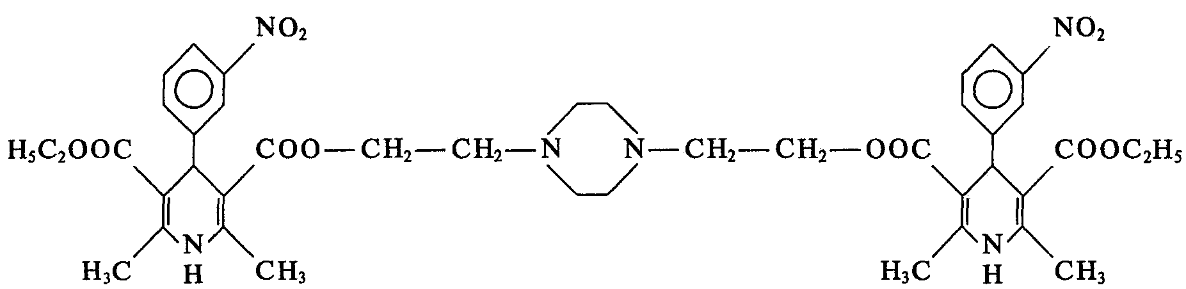

100 mmol 2,6-Dimethyl-5-ethoxycarbonyl-4-(3-nitrophenyl)-1,4-dihydropyridin-3-carbonsäure werden analog Beispiel 2 mit 50 mmol N,N'-Bis-(2-hydroxyethyl)-piperazin umgesetzt.
Schmelzpunkt: 203 bis 208°C; Ausbeute: 18%.

Beispiel 14

Bis-[2,6-dimethyl-5-ethoxycarbonyl-4-(3-nitrophenyl)-1,4-dihydropyridin-3-carbonsäure]-bis-hydroxyethylsulfid-diester

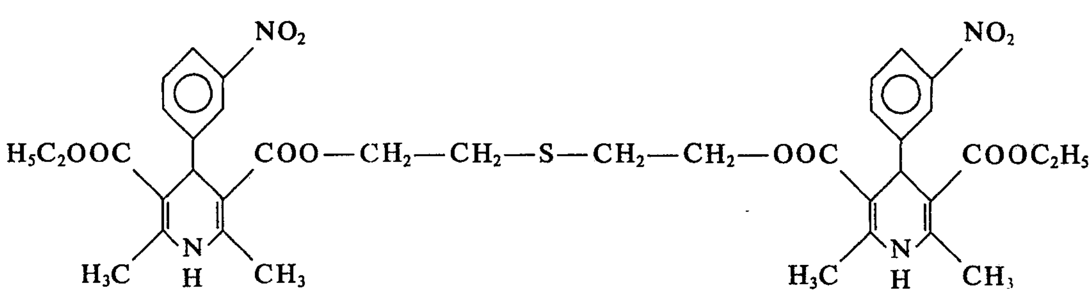

100 mmol 2,6-Dimethyl-5-ethoxycarbonyl-4-(2-nitrophenyl)-1,4-dihydropyridin-3-carbonsäure werden analog Beispiel 2 mit 50 mmol Bis-hydroxyethylsulfid umgesetzt.
Ausbeute: 70%.

Beispiel 15

Bis-[2,6-dimethyl-5-ethoxycarbonyl-4-(2-trifluormethylphenyl)-1,4-dihydropyridin-3-carbonsäure]-1,6-hexandiylester

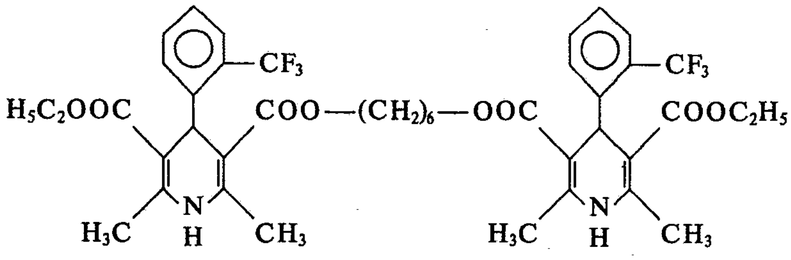

25 mmol Bis-(3-aminocrotonsäure)-1,6-hexandiylester werden analog Beispiel 3 mit 50 mmol 2-Trifluormethylbenzylidenacetessigsäureethylester umgesetzt.
Schmelzpunkt: 129—139°C; Ausbeute: 49%.

Beispiel 16

Bis-[2,6-dimethyl-5-(2-methoxy)-ethoxycarbonyl-4-(3-nitrophenyl)-1,4-dihydropyridin-3-carbonsäure]-1,6-hexandiylester

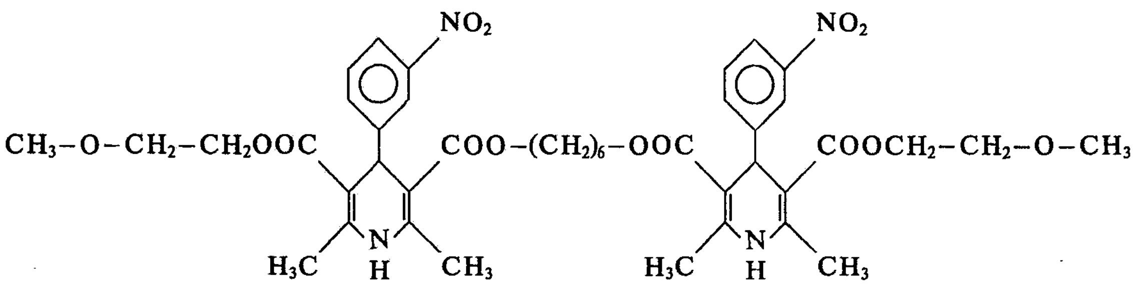

25 mmol Bis-(3-aminocrotonsäure)-1,6-hexandiylester werden analog Beispiel 3 mit 50 mmol 3-Nitrobenzylidenacetessigsäure-2-methoxyethylester umgesetzt.
Schmelzpunkt: 144—156°C; Ausbeute: 50%.

Beispiel 17

Bis-[2,6-dimethyl-5-ethoxycarbonyl-4-(3-nitrophenyl)-1,4-dihydropyridin-3-carbonsäure]-E-1,4-bis(hydroxymethyl)-cyclohexandiester

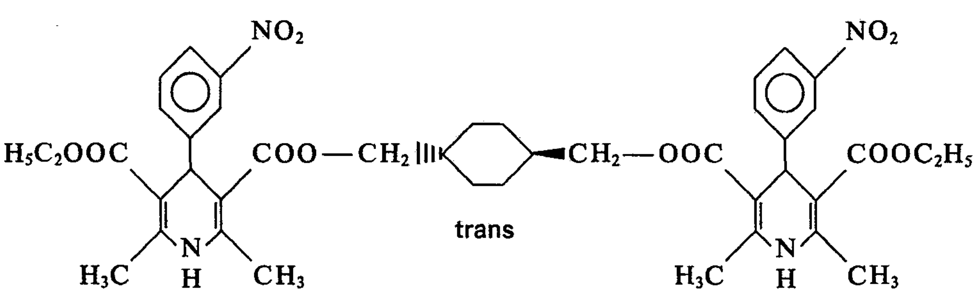

50 mmol 2,6-Dimethyl-5-ethoxycarbonyl-4-(3-nitrophenyl)-1,4-dihydropyridin-3-carbonsäure werden analog Beispiel 2 mit 25 mmol E-1,4-bis(hydroxymethyl)-cyclohexan umgesetzt.
Schmelzpunkt: 172—188°C; Ausbeute: 15%.

Beispiel 18

Bis-[2,6-dimethyl-5-ethoxycarbonyl-4-(3-nitrophenyl)-1,4-dihydropyridin-3-carbonsäure]-1,4-bis-(hydroxymethyl)-benzoldiester

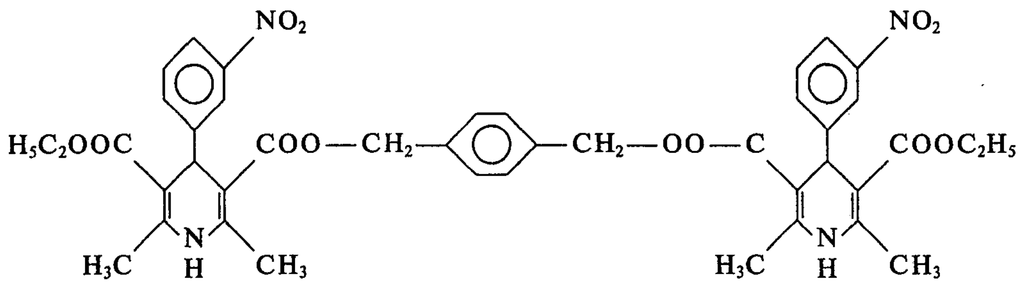

50 mmol 2,6-Dimethyl-5-ethoxycarbonyl-4-(3-nitrophenyl)-1,4-dihydropyridin-3-carbonsäure werden analog Beispiel 2 mit 25 mmol 1,4-Bis-(hydroxymethyl)-benzol umgesetzt.
Schmelzpunkt: 244 bis 259°C; Ausbeute: 30%.

Beispiel 19

Bis-[2,6-dimethyl-5-ethoxycarbonyl-4-(2-cyanophenyl)-1,4-dihydropyridin-3-carbonsäure]-1,6-hexandiylester

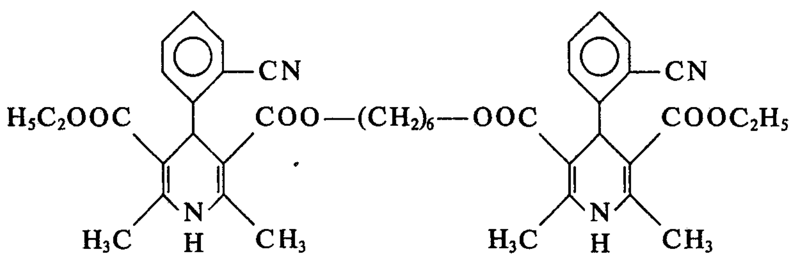

25 mmol Bis-(3-aminocrotonsäure)-1,6-hexandiylester werden mit 50 mmol 2-Cyanobenzylidenacetessigsäureethylester analog Beispiel 3 umgesetzt.
Ausbeute: 80%.

Beispiel 20

Bis-[2,6-dimethyl-5-ethoxycarbonyl-4-(3-nitrophenyl)-1,4-dihydropyridin-3-carbonsäure]-1,6-hexandiylamid

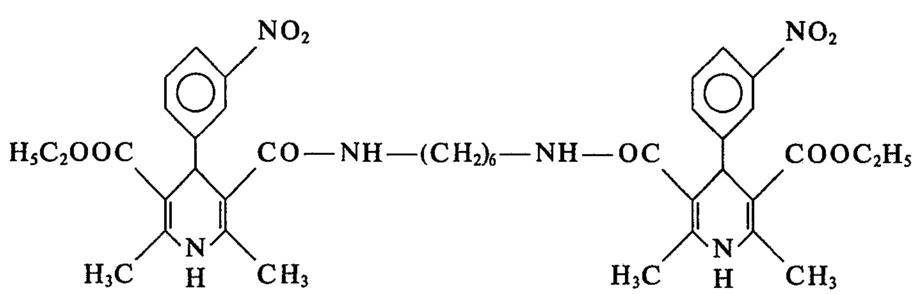

25 mmol 2,6-Dimethyl-5-ethoxycarbonyl-4-(3-nitrophenyl)-1,4-dihydropyridin-3-carbonsäure werden analog Beispiel 2 mit 12,5 mmol 1,6-Diaminohexan umgesetzt.
Ausbeute: 27%; (Fp.: 147–152°C).

Beispiel 21

Bis-[2,6-dimethyl-5-methoxycarbonyl-4-(2-chlorphenyl)-1,4-dihydropyridin-3-carbonsäure]-1,8-octandiylester

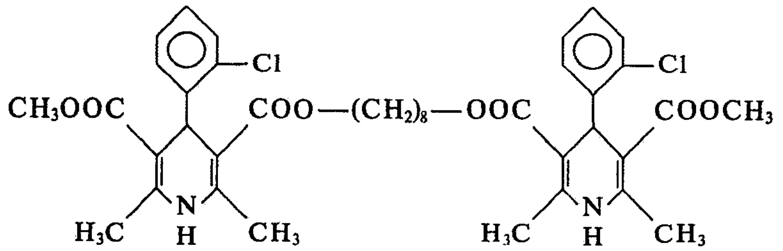

25 mmol Bis-(3-aminocrotonsäure)-1,6-hexandiylester werden mit 50 mmol 2-Chlorbenzylidenacetessigsäuremethylester analog Beispiel 3 umgesetzt.
Ausbeute: 80% amorphe Substanz.

$^1$H-NMR ($CDCl_3$):
$\delta$ = 1,0–1,4 (m, 8H), 1,4–1,7 (m, 4H), 2,2 (2s, 12H), 3,6 (s, 6H), 4,0 (t, 4H), 5,4 (s, 2H), 5,9 (s, NH), 6,9–7,5 (m, 8H).

Beispiel 22

Bis-[2,6-dimethyl-5-ethoxycarbonyl-4-(3-nitrophenyl)-1,4-dihydropyridin-3-carbonsäure]-N,N'-bishydroxyethylharnstoff-diester

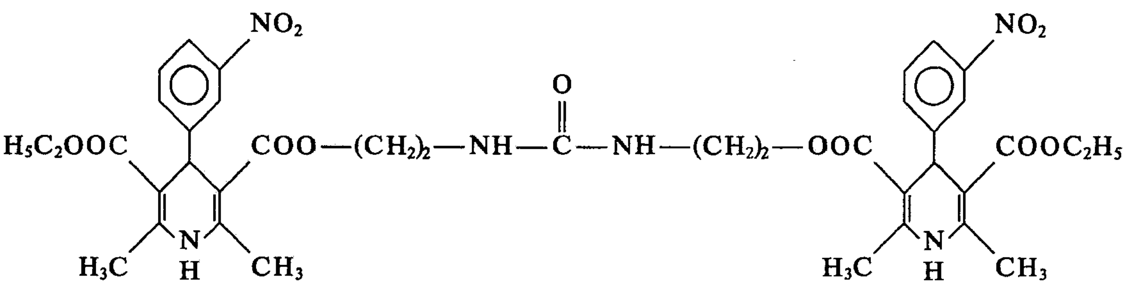

Darstellung analog Beispiel 2 aus N,N'-Bishydroxyethylharnstoff und 2,6-Dimethyl-5-ethoxycarbonyl-4-(3-nitrophenyl)-1,4-dihydropyridin-3-carbonsäure.
Ausbeute: 32%, Fp.: 178–187°C.

Beispiel 23

Bis-[2,6-dimethyl-5-methoxycarbonyl-4-(2-nitrophenyl)-1,4-dihydropyridin-3-carbonsäure]-bis-(2-hydroxyethyl)-sulfid-diester

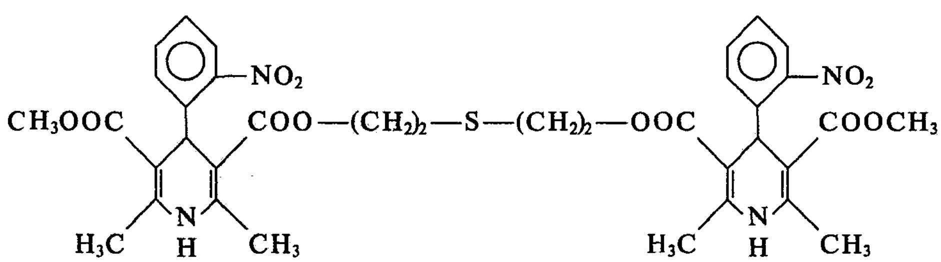

Darstellung analog Beispiel 3 aus Bis-(3-aminocrotonsäure)-bis-(2-hydroxyethyl)-sulfid-diester und 2-Nitrobenzylidenacetessigsäuremethylester.
Ausbeute: 80% amorphe Substanz.

$^1$H-NMR ($CDCl_3$):
$\delta$ = 2,3 (2s, 12H), 2,4–2,8 (m, 4H), 3,6 (s, 6H), 3,9–4,3 (m, 4H), 5,8 (s, 2H), 6,3 (s, NH), 7,1–7,8 (m, 8H).

Beispiel 24

Bis-[2,6-dimethyl-5-(2-methoxy)-ethoxycarbonyl-4-(2-chlorphenyl)-1,4-dihydropyridin-3-carbonsäure]-1,6-hexandiylester

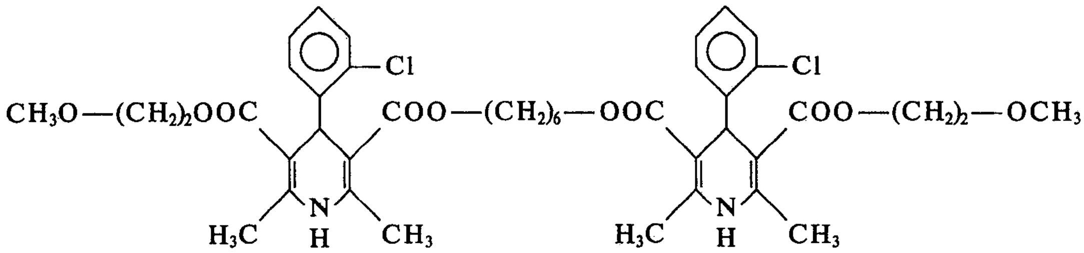

Darstellung analog Beispiel 3 aus 25 mmol Bis-(3-aminocrotonsäure)-1,6-hexandiylester und 50 mmol 2-Chlorbenzylidenacetessigsäure-2-methoxyethylester.
Ausbeute: 67% (Fp.: 135–153°C).

Beispiel 25

Bis-[2,6-dimethyl-5-ethoxycarbonyl-4-(2-trifluormethylphenyl)-1,4-dihydropyridin-3-carbonsäure]-bis-(2-hydroxyethyl)-sulfid-diester

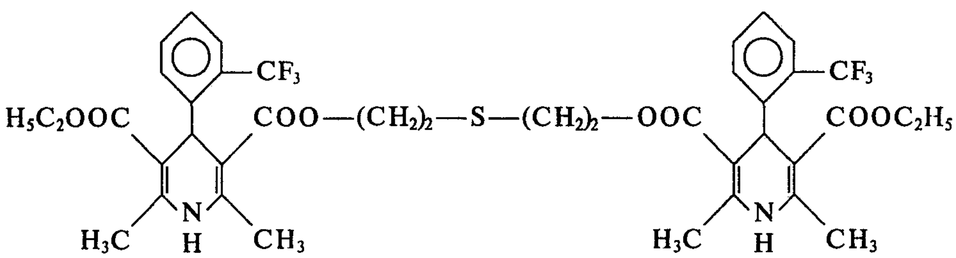

Darstellung analog Beispiel 3 aus 25 mmol Bis-(3-aminocrotonsäure)-bis-(2-hydroxyethyl)-sulfid-diester und 2-Trifluormethylbenzylidenacetessigsäuremethylester.
Ausbeute: 79% amorphe Substanz.

$^1$H-NMR ($CDCl_3$):
$\delta$ = 1,2 (t, 6H), 2,2 (s, 12H), 2,4–2,8 (m, 4H), 3,9–4,5 (m, 8H), 5,6 (s, 2H), 6,7 (s, NH), 7,1–7,7 (m, 8H).

Beispiel 26

Bis-[2,6-dimethyl-5-ethoxycarbonyl-4-(3-nitrophenyl)-1,4-dihydropyridin-3-carbonsäure]-bis-(2-hydroxyethyl)-disulfid-diester

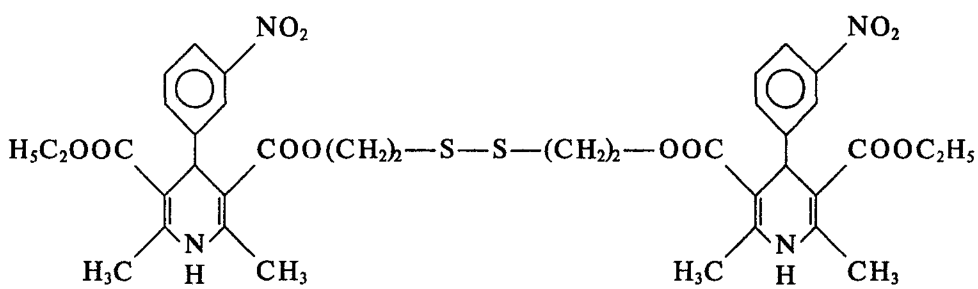

12,5 mmol Bis-(2-hydroxyethyl)-disulfid wurden analog Beispiel 2 mit 25 mmol 2,6-Dimethyl-5-ethoxycarbonyl-4-(3-nitrophenyl)-1,4-dihydropyridin-3-carbonsäure umgesetzt.
Ausbeute: 77% amorphe Substanz.

$^1$H-NMR ($CDCl_3$):
$\delta$ = 1,2 (t, 6H), 2,4 (s, 12H), 2,7–3,1 (m, 4H), 3,9–4,5 (m, 8H), 5,1 (s, 2H), 6,4 (s, NH), 7,2–8,2 (m, 8H).

Beispiel 27

Bis-[2,6-dimethyl-5-(2-methoxyethoxycarbonyl)-4-(3-nitrophenyl)-1,4-dihydropyridin-3-carbonsäure]-1,8-octandiylester

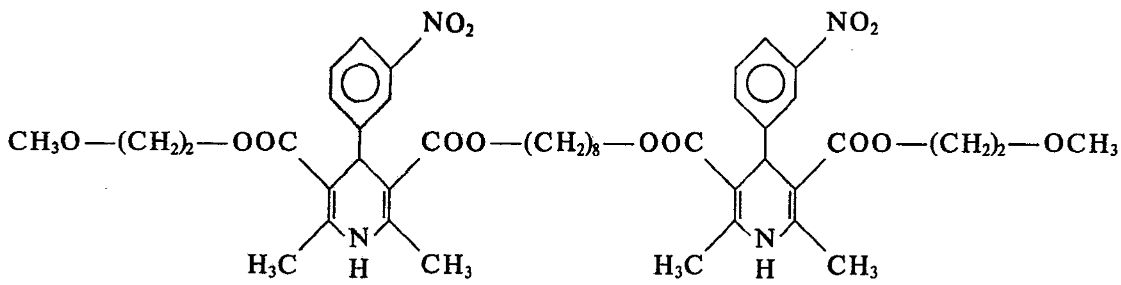

25 mmol Bis-(3-aminocrotonsäure)-1,8-octandiylester werden zusammen mit 50 mmol 3-Nitrobenzylidenacetessigsäure-2-methoxyethylester analog Beispiel 3 umgesetzt.
Ausbeute: 75% (Fp.: 146–150°C).

Beispiel 28

Bis-[2,6-dimethyl-5-(2-methoxyethoxycarbonyl-4-(3-nitrophenyl)-1,4-dihydropyridin-3-carbonsäure]-1,4-bis-(hydroxymethyl)-benzol-diester

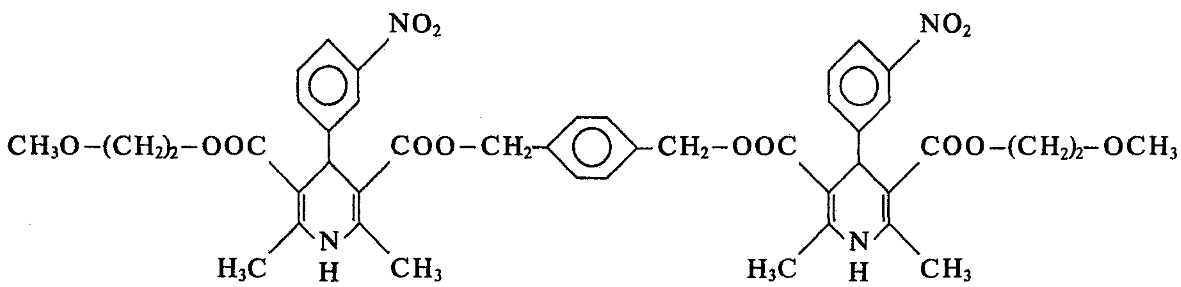

Darstellung analog Beispiel 3 aus 25 mmol Bis-(3-aminocrotonsäure)-1,4-bis-(hydroxymethyl)-benzol-diester und 50 mmol 3-Nitrobenzylidenacetessigsäure-2-methoxyethylester.
Ausbeute: 67% (Fp.: 159–162°C).

Beispiel 29

Bis-[2,6-dimethyl-5-(2-methoxyethoxycarbonyl-4-(2-trifluormethylphenyl)-1,4-dihydropyridin-3-carbonsäure]-bis-(2-hydroxyethyl)-sulfid-diester

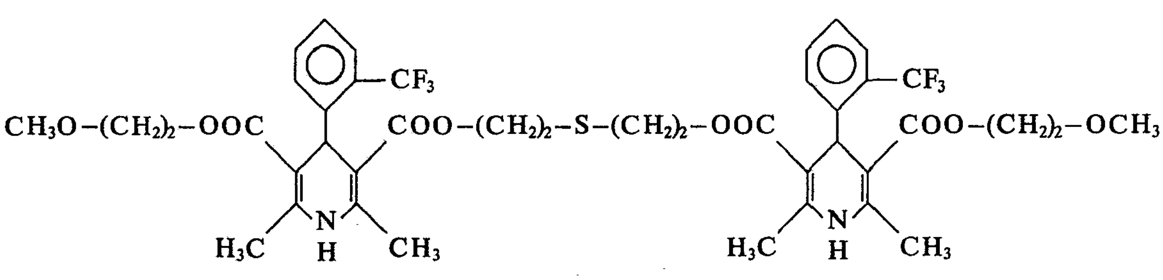

Darstellung analog Beispiel 3.
Ausbeute: 28% (Fp.: 100–112°C).

Beispiel 30

Bis-[2,6-dimethyl-5-methoxycarbonyl-4-(1-naphthyl)-1,4-dihydropyridin-3-carbonsäure]-1,8-octandiylester

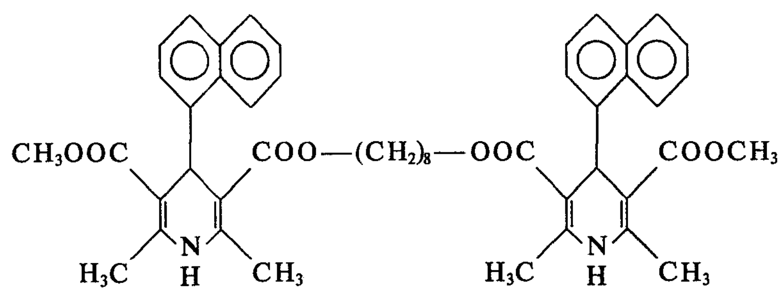

Darstellung analog Beispiel 3.
Ausbeute: 76% amorphe Substanz.

$^1$H-NMR ($CDCl_3$):
$\delta$ = 0,8–1,5 (m, 12H), 2,3 (2s, 12H), 3,5 (s, 6H), 3,6–4,1 (m, 4H), 5,8 (s, 2H), 6,1 (s, NH), 7,1–7,8 (m, 14H).

**Patentansprüche**

1. C-3 verknüpfte 1,4-Dihydropyridine der allgemeinen Formel I

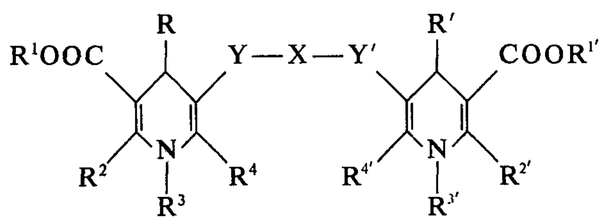

(I)

in welcher
R und R' gleich oder verschieden sind und jeweils für einen Phenylrest oder für einen Thienyl-, Furyl- oder Pyridylrest stehen, wobei der Phenylrest gegebenenfalls ein oder zwei gleiche oder verschiedene Substituenten aus der Gruppe Nitro, Cyano, Acido, Halogen, Trifluormethyl, Hydroxy, Amino, Alkyl, Alkoxy, Alkylamino oder Alkylmercapto mit jeweils 1 oder 2 Kohlenstoffatomen in den Alkylgruppen, substituiert ist,
$R^1$ und $R^{1'}$ gleich oder verschieden sind, jeweils für einen geradkettigen oder verzweigten Kohlenwasserstoffrest mit bis zu 6 Kohlenstoffatomen stehen, der gegebenenfalls durch ein Sauerstoffatom in der Kette unterbrochen ist und der gegebenenfalls substituiert ist durch Fluor, Chlor, Hydroxy, Phenyl oder Phenoxy.
$R^2$, $R^{2'}$, $R^4$ und $R^{4'}$ gleich oder verschieden sind und jeweils für Wasserstoff, geradkettigen oder verzweigten Alkylrest mit bis zu 4 Kohlenstoffatomen stehen, der gegebenenfalls durch Fluor, Chlor, Hydroxy, Phenyl oder Amino substituiert ist,
$R^3$ und $R^{3'}$ gleich oder verschieden sind und jeweils für Wasserstoff, Alkyl mit 1 bis 4 Kohlenstoffatomen, Phenyl, Benzyl oder Phenethyl, gegebenenfalls substituiert durch Hydroxy, Fluor oder Chlor,
Y und Y' jeweils gleich oder verschieden sind und die Bedeutung $-COO-$, $-CONH-$, $-CO-$ oder $-SO_2-$ haben, und
X ein Brückenglied bedeutet, das aus einer bis zehn $CH_2$-Gruppen als Kettenglied besteht, wobei keine dieser $CH_2$-Gruppen ein Ringglied sein darf, und wobei dieses Brückenglied zusätzlich in beliebiger Reihenfolge 1 bis 3 gleiche oder verschiedene Kettenglieder aus der Gruppe O, S, CO, CS, $NR^5$, $C(R^6)_2$, CH = N, Phenylen, Naphthylen, Pyrrylen, Cycloalkylen mit 5 bis 7 Kohlenstoffatomen, Piperazinylen, Piperidinylen, Pyrrolidinylen oder Morpholinylen enthalten kann, wobei $R^5$ für Wasserstoff, Benzyl oder Alkyl mit 1 bis 4 Kohlenstoffatomen steht und $R^6$ für Wasserstoff, Benzyl, Phenyl, Fluor, Chlor, Alkyl mit 1 bis 4 Kohlenstoffatomen, Hydroxy, Trifluormethyl, Cyano, Carboxy oder Amino steht,
sowie ihre pharmakologisch unbedenklichen Säureadditionssalze.

2. Verfahren zur Herstellung von C-3 verknüpften 1,4-Dihydropyridinen der allgemeinen Formel I

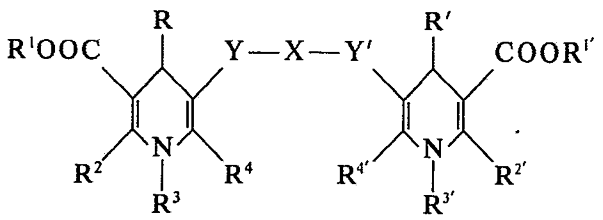

(I)

in welcher

R und R′ gleich oder verschieden sind und jeweils für einen Phenylrest oder für einen Thienyl-, Furyl- oder Pyridylrest stehen, wobei der Phenylrest gegebenenfalls ein oder zwei gleiche oder verschiedene Substituenten aus der Gruppe Nitro, Cyano, Acido, Halogen, Trifluormethyl, Hydroxy, Amino, Alkyl, Alkoxy, Alkylamino oder Alkylmercapto mit jeweils 1 oder 2 Kohlenstoffatomen in den Alkylgruppen, substituiert ist,

$R^1$ und $R^{1'}$ gleich oder verschieden sind jeweils für einen geradkettigen oder verzweigten Kohlenwasserstoffrest mit bis zu 6 Kohlenstoffatomen stehen, der gegebenenfalls durch ein Sauerstoffatom in der Kette unterbrochen ist und der gegebenenfalls substituiert ist durch Fluor, Chlor, Hydroxy, Phenyl oder Phenoxy,

$R^2$, $R^{2'}$, $R^4$ und $R^{4'}$ gleich oder verschieden sind und jeweils für Wasserstoff, geradkettigen oder verzweigten Alkylrest mit bis zu 4 Kohlenstoffatomen stehen, der gegebenenfalls durch Fluor, Chlor, Hydroxy, Phenyl oder Amino substituiert ist,

$R^3$ und $R^{3'}$ gleich oder verschieden sind und jeweils für Wasserstoff, Alkyl mit 1 bis 4 Kohlenstoffatomen, Phenyl, Benzyl oder Phenethyl, gegebenenfalls substituiert durch Hydroxy, Fluor oder Chlor,

Y und Y′ jeweils gleich oder verschieden sind und die Bedeutung $-COO-$, $-CONH-$, $-CO-$ oder $-SO_2-$ haben, und

X ein Brückenglied bedeutet, das aus einer bis zehn $CH_2$-Gruppen als Kettenglied besteht, wobei keine dieser $CH_2$-Gruppen ein Ringglied sein darf, und wobei dieses Brückenglied zusätzlich in beliebiger Reihenfolge 1 bis 3 gleiche oder verschiedene Kettenglieder aus der Gruppe O, S, CO, CS, $NR^5$, $C(R^6)_2$, $CH=N$, Phenylen, Naphthylen, Pyrrylen, Cycloalkylen mit 5 bis 7 Kohlenstoffatomen, Piperazinylen, Piperidinylen, Pyrrolidinylen oder Morpholinylen enthalten kann, wobei $R^5$ für Wasserstoff, Benzyl oder Alkyl mit 1 bis 4 Kohlenstoffatomen steht und $R^6$ für Wasserstoff, Benzyl, Phenyl, Fluor, Chlor, Alkyl mit 1 bis 4 Kohlenstoffatomen, Hydroxy, Trifluormethyl, Cyano, Carboxy oder Amino steht,

sowie ihre pharmakologisch unbedenklichen Säureadditionssalze,

dadurch gekennzeichnet, daß man

A) 1,4-Dihydropyridinhydroxyderivate der allgemeinen Formel II

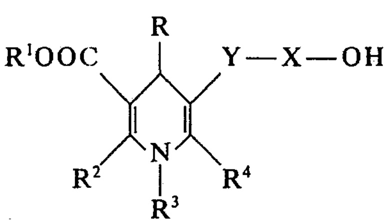

(II)

in welcher

R, $R^1$, $R^2$, $R^3$, $R^4$, Y und X die oben angegebene Bedeutung haben,

in einem inerten organischen Lösungsmittel in Gegenwart von wasserbindenden Mitteln mit 1,4-Dihydropyridin-3-carbonsäurederivaten der allgemeinen Formel III

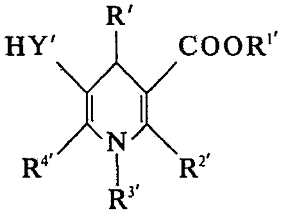

(III)

in welcher

R′, $R^{1'}$, $R^{2'}$, $R^{3'}$, $R^{4'}$ und Y′ die oben angegebene Bedeutung besitzen, wobei Y′ nicht für eine Carbonylgruppe steht,

in äquivalenten Mengen unter Wasserabspaltung bei Temperaturen zwischen 0 und 180°C umsetzt, oder

B) 1,4-Dihydropyridincarbonsäuren der allgemeinen Formel IV

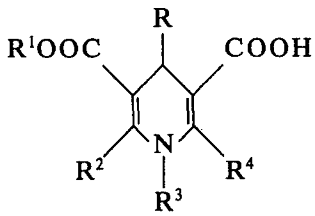

(IV)

in welcher
R, $R^1$, $R^2$, $R^3$ und $R^4$ die oben angegebene Bedeutung haben,
in Gegenwart eines inerten organischen Lösungsmittels und gegebenenfalls in Gegenwart eines Kondensationsmittels mit einer bifunktionellen Verbindung der allgemeinen Formel V

$$Z—X—Z' \quad (V)$$

in welcher
X die oben angegebene Bedeutung hat und
Z und Z' jeweils gleich oder verschieden sind und für Hydroxyl, Mercapto oder den Rest $NR^5H$ stehen, wobei $R^5$ die oben angegebene Bedeutung besitzt,
in einem molaren Verhältnis von etwa 2:1 bei Temperaturen zwischen 0 und 180°C umsetzt, wobei nach dieser Variante nur Verbindungen der allgemeinen Formel I erhalten werden, in denen Y und Y' nicht für eine Carbonylgruppe stehen, oder

C) Yliden-β-ketoester der allgemeinen Formel VI

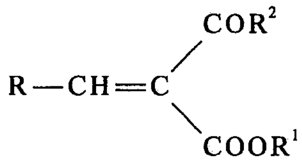

(VI)

R, $R^1$ und $R^2$ die oben angegebene Bedeutung haben,
mit Enaminocarbonsäureestern der allgemeinen Formel VII

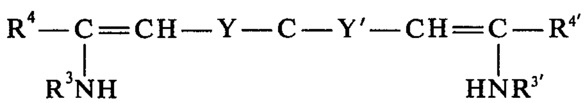

(VII)

in welcher
$R^3$, $R^{3'}$, $R^4$, $R^{4'}$, Y und Y' die oben angegebene Bedeutung haben,
in Gegenwart von inerten organischen Lösungsmitteln in molaren Verhältnissen von etwa 2:1 bei Temperaturen zwischen 0 und 180°C umsetzt.

3. Verfahren gemäß Anspruch 2, dadurch gekennzeichnet, daß man die Umsetzung bei Temperaturen zwischen 20 und 120°C durchführt.
4. Verbindungen gemäß Anspruch 1 zur Verwendung bei der Bekämpfung von Erkrankungen.
5. Verbindungen gemäß Anspruch 1 zur Verwendung bei der Bekämpfung von Kreislauferkrankungen.
6. Arzneimittel enthaltend mindestens eine Verbindung der allgemeinen Formel I gemäß Anspruch 2.
7. Verfahren zur Herstellung von Arzneimitteln, dadurch gekennzeichnet, daß man mindestens eine Verbindung der allgemeinen Formel I gemäß Anspruch 2 gegebenenfalls unter Zusatz von Hilfs- und Trägerstoffen in eine geeignete Applikationsform überführt.

**Claims**

1. 1,4-Dihydropyridines which are linked in the C-3 position, of the general formula I

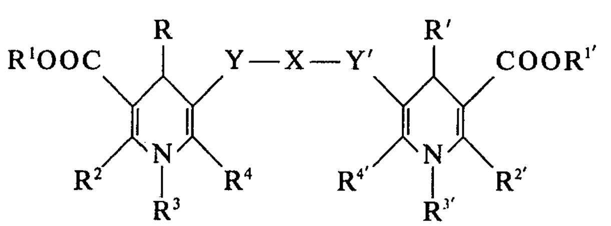

(I)

in which
R and R' are identical or different and each represent a phenyl radical or a thienyl, furyl, or pyridyl radical, the phenyl radical is optionally substituted by one or two identical or different substituents from the group comprising nitro, cyano, azido, halogen, trifluoromethyl, hydroxyl, amino, alkyl, alkoxy, alkylamino or alkylmercapto, with in each case 1 or 2 carbon atoms in the alkyl groups,
$R^1$ and $R^{1'}$ are identical or different and each represent a straight-chain or branched hydrocarbon radical having up to 6 carbon atoms which is optionally interrupted in the chain by an oxygen atom and which is optionally substituted by fluorine, chlorine, hydroxyl, phenyl or phenoxy,
$R^2$, $R^{2'}$, $R^4$ and $R^{4'}$ are identical or different and each represent hydrogen or a straight-chain or branched alkyl radical which has up to 4 carbon atoms and which is optionally substituted by fluorine, chlorine, hydroxyl, phenyl or amino,
$R^3$ and $R^{3'}$ are identical or different and each represent hydrogen, alkyl having 1 to 4 carbon atoms, phenyl, benzyl or phenethyl, optionally substituted by hydroxyl, fluorine or chlorine,
Y and Y' are in each case identical or different and each denote

$-COO-$, $-CONH-$, $-CO-$ or $-SO_2-$ and

X denotes a bridge member which consists of one to ten $CH_2$ groups as the chain member, and none of these $CH_2$ groups are allowed as a ring member, it being possible for this bridge member additionally to contain, in any sequence, 1 to 3 identical or different chain members from the group comprising O, S, CO, CS, $NR^5$, $C(R^6)_2$, CH = N, phenylene, naphthylene, pyrrylene, cycloalkylene with 5 to 7 carbon atoms, piperazinylene, piperidinylene, pyrrolidinylene or morpholinylene,
wherein
$R^5$ represents hydrogen, benzyl or alkyl having 1 to 4 carbon atoms and $R^6$ represents hydrogen, benzyl, phenyl, fluorine, chlorine, alkyl having 1 to 4 carbon atoms, hydroxyl, trifluoromethyl, cyano, carboxyl or amino
and their pharmacologically acceptable acid addition salts.

2. Process for the preparation of 1,4-dihydropyridines which are linked in the C-3 position, of the general formula I

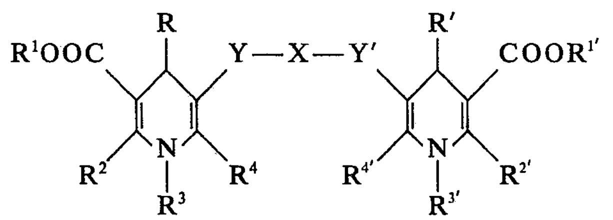

(I)

in which
R and R' are identical or different and each represent a phenyl radical or a thienyl, furyl, or pyridyl radical, the phenyl radical is optionally substituted by one or two identical or different substituents from the group comprising nitro, cyano, azido, halogen, trifluoromethyl, hydroxyl, amino, alkyl, alkoxy, alkylamino or alkylmercapto, with in each case 1 or 2 carbon atoms in the alkyl groups,
$R^1$ and $R^{1'}$ are identical or different and each represent a straight-chain or branched hydrocarbon radical having up to 6 carbon atoms which is optionally interrupted in the chain by an oxygen atom and which is optionally substituted by fluorine, chlorine, hydroxyl, phenyl or phenoxy,
$R^2$, $R^{2'}$, $R^4$ and $R^{4'}$ are identical or different and each represent hydrogen or a straight-chain or branched alkyl radical which has up to 4 carbon atoms and which is optionally substituted by fluorine, chlorine, hydroxyl, phenyl or amino,
$R^3$ and $R^{3'}$ are identical or different and each represent hydrogen, alkyl having 1 to 4 carbon atoms, phenyl, benzyl or phenethyl, optionally substituted by hydroxyl, fluorine or chlorine,
Y and Y' are in each case identical or different and each denote

$-COO-$, $-CONH-$, $-CO-$ or $-SO_2-$ and

X denotes a bridge member which consists of one to ten $CH_2$ groups as the chain member, and none of these $CH_2$ groups are allowed as a ring member, it being possible for this bridge member additionally to contain, in any sequence, 1 to 3 identical or different chain members from the group comprising O, S, CO, CS, $NR^5$, $C(R^6)_2$, CH = N, phenylene, naphthylene, pyrrylene, cycloalkylene with 5 to 7 carbon atoms, piperazinylene, piperidinylene, pyrrolidinylene or morpholinylene,
wherein
$R^5$ represents hydrogen, benzyl or alkyl having 1 to 4 carbon atoms and $R^6$ represents hydrogen, benzyl, phenyl, fluorine, chlorine, alkyl having 1 to 4 carbon atoms, hydroxyl, trifluoromethyl, cyano, carboxyl or amino
and their pharmacologically acceptable acid addition salts, characterised in that

A) hydroxy-1,4-dihydropyridine derivatives of the general formula II

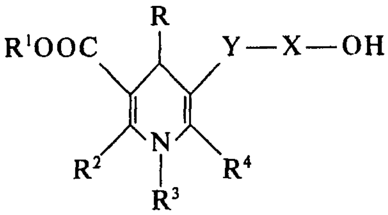

(II)

in which
R, $R^1$, $R^2$, $R^3$, $R^4$, Y and X have the abovementioned meaning,
are reacted with equivalent amounts of 1,4-dihydropyridine-3-carboxylic acid derivatives of the general formula III

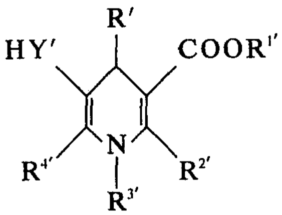

(III)

in which
R', $R^{1'}$, $R^{2'}$, $R^{3'}$, $R^{4'}$ and Y' have the abovementioned meaning, but Y' does not represent a carbonyl group,
in an inert organic solvent in the presence of waterbinding agents at temperatures between 0 and 180°C, water being split off, or

B) 1,4-dihydropyridinecarboxylic acids of the general formula IV

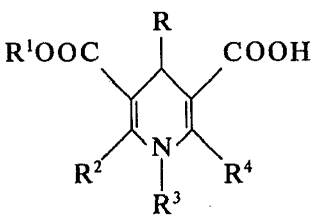

(IV)

in which
R, $R^1$, $R^2$, $R^3$ and $R^4$ have the abovementioned meaning,
are reacted with a bifunctional compound of the general formula V

Z—X—Z' (V)

in which
X has the abovementioned meaning and
Z and Z' are in each case identical or different and represent hydroxyl, mercapto or the radical $NR^5H$,
wherein
$R^5$ has the abovementioned meaning,
in a molar ratio of about 2:1 in the presence of an inert organic solvent and, if appropriate, in the presence of a condensation agent, at temperatures between 0 and 180°C, only compounds of the general formula I in which
Y and Y' do not represent a carbonyl group being obtained by this variant, or

C) ylidene-$\beta$-keto esters of the general formula VI

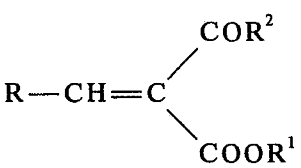

(VI)

R, $R^1$ and $R^2$ have the abovementioned meaning, are reacted with enaminocarboxylic acid esters of the general formula VII

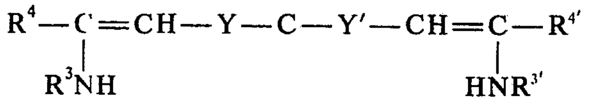

(VII)

in which
$R^3$, $R^{3'}$, $R^4$, $R^{4'}$, Y and Y' have the abovementioned meaning,
in molar ratios of about 2:1 in the presence of inert organic solvents at temperatures between 0 and 180°C.

3. Process according to Claim 2, characterised in that the reaction is carried out at temperatures between 20 and 120°C.
4. Compounds according to Claim 1 for use in combating illnesses.
5. Compounds according to Claim 1 for use in combating circulatory illnesses.
6. Medicaments containing at least one compound of the general formula I according to Claim 2.
7. Process for the preparation of medicaments, characterised in that at least one compound of the general formula I according to Claim 2 is converted into a suitable form for administration, if necessary with the addition of auxiliaries and excipients.

**Revendications**

1. 1,4-Dihydropyridines reliées par les atomes de carbone en position 3, de foumule générale I:

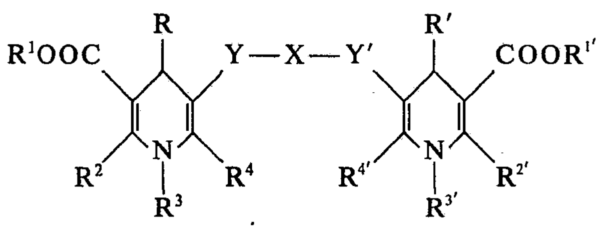

(I)

dans laquelle
R et R' sont identiques ou différents et représentent chacun un radical phényle ou un radical thiényle, furyle ou pyridyle, le radical phényle portant éventuellement un ou deux substituants identiques ou différents choisis dans l'ensemble constitué par un groupe nitro, cyano, acido, halogéno, trifluorométhyle, hydroxyle, amino, alkyle, alcoxy, alkylamino ou alkylmercapto ayant à chaque fois 1 ou 2 atomes de carbone dans les groupes alkyles,
$R^1$ et $R^{1'}$ sont identiques ou différents et représentent chaque fois un radical d'hydrocarbure linéaire ou ramifié ayant 1 à 6 atomes de carbone, dont la chaîne peut être interrompue éventuellement par un atome d'oxygène et qui est éventuellement substitué par du fluor, du chlore, un groupe hydroxyle, phényle ou phénoxy,
$R^2$, $R^{2'}$, $R^4$ et $R^{4'}$ sont identiques ou différents et représentent chacun un atome d'hydrogène, un radical alkyle linéaire ou ramifié ayant 1 à 4 atomes de carbone, qui est éventuellement substitué par du fluor, du chlore, un groupe hydroxyle, phényle ou amino,
$R^3$ et $R^{3'}$ sont identiques ou différents et représentent chacun un atome d'hydrogène, un groupe alkyle ayant 1 à 4 atomes de carbone, phényle, benzyle ou phénétyle, éventuellement substitués par un groupe hydroxyle, par du fluor ou du chlore,
Y et Y' sont identiques ou différents et représentent $-COO-$, $-CONH-$, $-CO-$ ou $-SO_2-$, et
X est une chaîne de pontage, qui consiste en un à dix groupes $CH_2$ comme chaînon(s), aucun de ces groupes $CH_2$ ne devant être un chaînon cyclique, et cette chaîne de pontage pouvant en outre contenir, dans un ordre quelconque, 1 à 3 chaînons identiques ou différents choisis dans l'ensemble constitué par O, S, CO, CS, $NR^5$, $C(R^6)_2$, $CH=N$, un groupe phénylène, naphtylène, pyrrylène, cycloalkylène ayant 5 à 7 atomes de carbone, pipérazinylène, pipéridinylène, pyrrolidinylène ou morpholinylène, où $R^5$ représente un atome d'hydrogène, un groupe benzyle ou alkyle ayant 1 à 4 atomes de carbone et $R^6$ représente un atome d'hydrogène, un groupe benzyle, phényle, fluor, chlore, alkyle ayant 1 à 4 atomes de carbone, hydroxyle, trifluorométhyle, cyano, carboxy ou amino,
ainsi que leurs sels d'addition d'acides sans inconvénient du point de vue physiologique.

2. Procédé de préparation 1,4-dihydropyridines, reliées par les atomes de carbone en position 3, de foumule générale I:

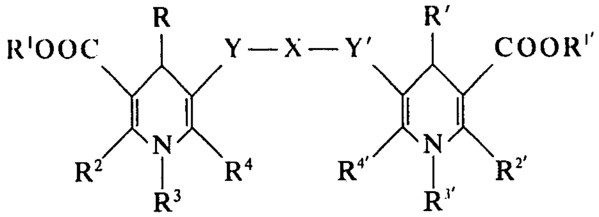

(I)

dans laquelle
R et R' sont identiques ou différents et représentent chacun un radical phényle ou un radical thiényle, furyle ou pyridyle, le radical phényle portant éventuellement un ou deux substituants identiques ou différents choisis dans l'ensemble constitué par un group nitro, cyano, acido, halogéno, trifluorométhyle, hydroxyle, amino, alkyle, alcoxy, alkylamino ou alkylmercapto ayant chacun 1 ou 2 atomes de carbone dans les groupes alkyles,
$R^1$ et $R^{1'}$ sont identiques ou différents et représentent chacun un radical d'hydrocarbure linéaire ou ramifié ayant jusqu'à 6 atomes de carbone, dont la chaîne peut être éventuellement interrompue par un atome d'oxygène et qui est éventuellement substitué par du fluor, du chlore, un groupe hydroxyle, phényle ou phénoxy,
$R^2$, $R^{2'}$, $R^4$ et $R^{4'}$ sont identiques ou différents et représentent chacun un atome d'hydrogène, un radical alkyle linéaire ou ramifié ayant jusqu'à 4 atomes de carbone, et qui est éventuellement substitué par du fluor, du chlore, un groupe hydroxyle, phényle ou amino,
$R^3$ et $R^{3'}$ sont identiques ou différents et représentent chacun un atome d'hydrogène, un groupe alkyle ayant 1 à 4 atomes de carbone, un groupe phényle, benzyle ou phénéthyle, éventuellement substitué par un groupe hydroxyle, par du fluor ou du chlore,
Y et Y' sont identiques ou différents et représentent chacun $-COO-$, $-CONH-$, $-CO-$ ou $-SO_2-$, et X représente une chaîne de pontage, qui consiste en un à dix groupes $CH_2$ comme chaînons, aucun de ces groupes $CH_2$ ne devant être un chaînon cyclique, cette chaîne de pontage pouvant contenir en outre dans un ordre quelconque, un à trois chaînons identiques ou différents choisis parmi O, S, CO, CS, $NR^5$, $C(R^6)_2$, $CH=N$, un groupe phénylène, naphtylène, pyrrylène, cycloalkylène ayant 5 à 7 atomes de carbone, pipérazinylène, pipéridinylène, pyrrolidinylène ou morpholinylène, où $R^5$ représente un atome d'hydrogène ou un groupe benzyle ou alkyle ayant 1 à 4 atomes de carbone et $R^6$ représente un atome d'hydrogène, un groupe benzyle, phényle, un atome de fluor ou de chlore, un groupe alkyle ayant 1 à 4 atomes de carbone, hydroxyle, trifluorométhyle, cyano, carboxy ou amino,
ainsi que leurs sels d'addition d'acides sans inconvénient du point de vue pharmacologique,
procédé caractérisé en ce que:

A) on fait réagir, en des quantités équivalentes avec séparation de l'eau, à des températures comprises entre 0 et 180°C, des dérivés hydroxylés de la 1,4-dihydropyridine de formule générale II:

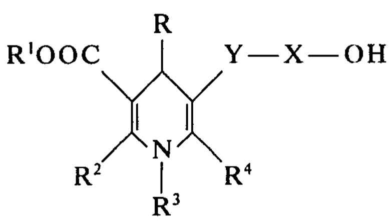

(II)

dans laquelle
R, $R^1$, $R^2$, $R^3$, $R^4$, Y et X ont le sens indiqué ci-dessus,
dans un solvant organique inerte, en présence d'agent de fixation d'eau, avec des dérivés de l'acide 1,4-dihydropyridine-3-carboxylique de formule générale III:

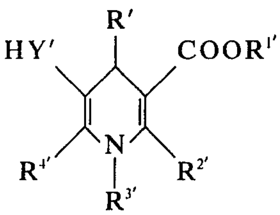

(III)

dans laquelle
$R'$, $R^{1'}$, $R^{2'}$, $R^{3'}$, $R^{4'}$ et Y' ont le sens indiqué ci-dessus, Y' ne devant pas représenter un groupe carbonyle, ou

B) on fait réagir, selon un rapport molaire d'environ 2:1, à des températures comprises entre 0 et 180°C, des acides 1,4-dihydropyridinecarboxyliques de formule générale IV:

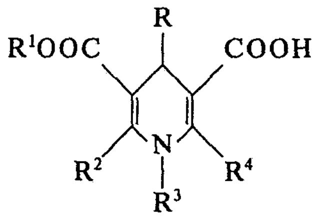

(IV)

dans laquelle
R, $R^1$, $R^2$, $R^3$ et $R^4$ ont le sens indiqué ci-dessus,
en présence d'un solvant organique inerte et éventuellement en présence d'un agent de condensation, avec un composé bifonctionnel de formule générale V:

$$Z—X—Z' \qquad (V)$$

dans laquelle
X a le sens indiqué ci-dessus, et
Z et Z' sont identiques ou différents et représentent chacun un groupe hydroxyle, mercapto ou le radical $NR^5H$, où $R^5$ a le sens indiqué ci-dessus,
et, dans cette variante, on n'obtient que des composés de formule générale I, dans lesquels Y et Y' ne représentent pas un groupe carbonyle, ou

C) on fait réagir selon des rapports molaires d'environ 2:1, à des températures comprises entre 0 et 180°C, en présence de solvants organiques inertes, des ilydène-β-cétoesters de formule générale VI:

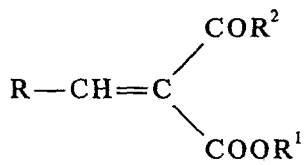

(VI)

dans laquelle
R, $R^1$ et $R^2$ ont le sens indiqué ci-dessus,
avec des esters d'acides énaminocarboxyliques de formule générale VII:

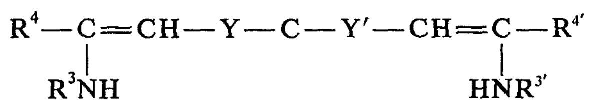

(VII)

dans laquelle
$R^3$, $R^{3'}$, $R^4$, $R^{4'}$, Y et Y' ont le sens indiqué ci-dessus.

3. Procédé selon la revendication 2, caractérisé en ce qu'on effectue la réaction à des températures comprises entre 20 et 120°C.

4. Composés selon la revendication 1, destinés à servir à combattre des maladies.

5. Composés selon la revendication 1, destinés à combattre les maladies de la circulation sanguine.

6. Médicament contenant au moins un composé de formule générale I selon la revendication 2.

7. Procédé de préparation de médicaments, caractérisé en ce qu'on transforme au moins un composé de formule générale I selon la revendication 2, éventuellement avec addition d'adjuvants et de supports, en une forme convenant pour leur administration.